# EUROPEAN PATENT SPECIFICATION

(11) **EP 1 592 347 B1**
(45) Date of publication and mention of the grant of the patent: **21.04.2010**
(21) Application number: 03704982.2
(22) Date of filing: 05.02.2003
(51) Int. Cl.: A61B 6/03

(54) **DUAL FUNCTION CT SCAN**
ZWEIFACH-FUNKTIONS CT-SCAN
TOMODENSITOMETRE A DOUBLE FONCTION

(43) Date of publication of application: 09.11.2005
(73) Proprietor: Koninklijke Philips Electronics N.V., 5621 BA Eindhoven (NL)
(72) Inventor: MARKOVITCH, Armin, 26367 Kiryat Motzkin (IL)
(74) Representative: Volmer, Georg
(86) International application number: PCT/IL2003/000093
(87) International publication number: WO 2004/069053

(56) References cited:
- US-A- 5 457 724
- US-A- 5 608 772
- US-A- 5 822 393

## Description

### FIELD OF THE INVENTION

The present invention relates to computerized tomography (CT) X-ray imaging, and in particular to methods of improving efficiency of CT scans.

### BACKGROUND OF THE INVENTION

In CT X-ray imaging of a patient, X-rays are used to image internal structure and features of a region of interest (ROI) of the patient's body. The imaging is performed by a CT scanner comprising an X-ray source that provides a fan-shaped X-ray beam if the scanner is a single slice scanner or, if the scanner is a multislice scanner, a cone-shaped X-ray beam. An array of closely spaced X-ray detectors positioned facing the X-ray source receives X-rays from the X-ray source that pass through the patient's body. The X-ray source and detector array are mounted in a gantry and the patient is supported on an appropriate support couch. The couch is moveable axially, along an axis referred to as a "z-axis", relative to the gantry and the gantry, or X-ray source supported in the gantry, is rotatable around the z-axis.

To image the ROI, the couch is moved along the z-axis to translate the ROI through a field of view (FOV) of the scanner, which is located between the scanner's X-ray source and detector array. As the ROI moves through the FOV the X-ray source is rotated around the z-axis to illuminate the ROI with X-rays at a plurality of different view angles. At each view angle and different axial positions of the ROI detectors in the array of detectors measure intensity of X-rays from the source that pass through the ROI. The intensity of X-rays measured by a given detector in the array of detectors is a function of an amount by which X-rays are attenuated by material in the slice along a path length from the X-ray source, through the ROI to the given detector. The measurement provides information on composition and density of tissue in the ROI along the path-length.

In some CT scanners an axial scan of a patient is performed in which the patient is moved stepwise along the z-axis to "step" the ROI through the FOV. Following each step, the X-ray source is rotated through 360 degrees or about 180 degrees to acquire attenuation measurements for the ROI. In some CT scanners a "spiral scan" of a patient is performed in which the patient is steadily advanced through the gantry while the X-ray source simultaneously rotates around the patient and projections of slices in the region are acquired "on the fly".

The attenuation measurements for an ROI of a patient provided by the detectors in an axial or spiral scan of the patient are processed using algorithms known in the art to provide a map of the absorption coefficient of material in the ROI as a function of position. The map is used to display and identify internal organs and features of the region.

Generally, prior to a CT scanner being controlled to perform an "imaging scan" of an ROI of a patient's body, the scanner is controlled to perform at least one "reconnaissance scan" of the patient. The reconnaissance scan provides an image of the morphology of the patient's body, which is used to identify a location of the ROI in the body relative to features of the body structure and/or relative to a CT scanner coordinate system. The location of the ROI is used to plan the imaging scan and in particular to determine where to begin and end an imaging scan of the patient. A reconnaissance scan is usually a planar scan in which a patient is imaged at a constant view angle.

### SUMMARY OF THE INVENTION

An aspect of some embodiments of the present invention, as defined by independent claims 1 and 20, relates to providing a CT scanner controllable to perform a scan of a patient that incorporates a CT reconnaissance scan and a CT imaging scan. As a result, instead of the CT scanner performing two scans, a reconnaissance scan and then an imaging scan, to properly image an ROI of a patient, the scanner may image the ROI using a single "dual function" scan.

By performing a dual function scan, in accordance with an embodiment of the present invention, an ROI of a patient can generally be imaged in less time than in prior art. In addition, to an extent that in prior art an ROI of the patient is exposed to radiation from both a reconnaissance and an imaging scan, a dual function scan may reduce an amount of radiation to which the patient is exposed to image the ROI.

In accordance with an embodiment of the present invention, as a patient is moved along the z-axis through the FOV of a CT scanner, at a position of the couch before the ROI enters the FOV of the scanner, the scanner begins to scan the patient in a reconnaissance mode with optionally low intensity X-rays. The position at which a reconnaissance mode scan begins is hereinafter referred to as a "reconnaissance start position". As the low intensity reconnaissance mode scan progresses, scan data, *i.e*. attenuation data, is used to generate an image of the scanned portion of the patient's body. An image generated by the scanner of a portion of a patient's body using data from a reconnaissance mode scan is referred to as a "reconnaissance image". A location of at least one feature identified in the optionally low intensity reconnaissance image is used to estimate a location of the ROI. The estimated location of the ROI and a speed at which the patient moves through the scanner FOV are used to estimate at which position of the couch, when during the scan, the ROI is expected to enter the scanner's FOV.

At a position shortly before the expected arrival of the ROI in the field of view, the scanner adjusts intensity of X-rays to an intensity required to acquire attenuation data appropriate for imaging the ROI and continues the scan in an "imaging mode" to acquire sufficient data to image the ROI. Generally, adjusting the X-ray intensity involves increasing the intensity from the relatively low intensity X-rays used during the reconnaissance scan. The position at which the relatively high-intensity imaging scan of the ROI begins is hereinafter referred to as an "imaging start position".

In some embodiments of the present invention, a controller comprised in the CT scanner processes the low-intensity data to determine an imaging start position and at the imaging start position increases intensity of X-rays from the X-ray source automatically.

In some embodiments of the present invention, the controller generates an image of the scanned portion of the patient's body on a video console as the low-intensity scan progresses and an operator determines from the generated image an imaging start position at which to increase X-ray intensity. In some embodiments of the present invention the controller, while not by itself controlling the X-ray source to increase X-ray intensity at an imaging start position does generate cues to aid an operator in determining when to begin a high intensity imaging portion of the dual function scan. Such cues may be visual and/or aural and may, for example, draw the operator's attention to note that a particular feature of the patient's anatomy is entering the scanner's FOV and that within a certain predetermined period of time the ROI is to enter the FOV.

Data acquired during the high intensity scan and/or the preceding low intensity scan are used to predict a position of the couch, hereinafter an "exit position" at which the ROI is expected to exit the scanner's FOV. At a couch position, hereinafter an "imaging stop position", that occurs shortly after a time at which the ROI exits the FOV, the scanner optionally turn off the X-ray source, in which case the imaging stop position coincides with a "scan termination position". Alternatively, at the imaging stop position the scanner may lower intensity of X-rays provided by the X-ray source and continue scanning in a reconnaissance mode for a relatively short period of time at low intensity. Attenuation data provided by the low intensity reconnaissance mode scan following the relatively high intensity imaging mode scan is optionally used to assure that all of the ROI is scanned properly. In some embodiments of the present invention, data from a reconnaissance mode scan performed prior to and optionally after the imaging mode scan is used to provide a background image over which an image of the ROI generated from the imaging scan data is displayed.

Generally a reconnaissance mode scan of a dual function scan, in accordance with an embodiment of the present invention, is an axial or helical mode scan respectively if the imaging mode scan is an axial or helical mode scan. However, a reconnaissance mode scan of a dual function scan may be an axial mode scan independent of whether the imaging mode scan is an axial or helical mode scan.

There is therefore provided in accordance with an embodiment of the present invention, a CT scanner for imaging an ROI of a patient comprising: an X-ray source and detector array that define an FOV of the scanner; a couch on which the patient is supported that moves the patient through the FOV; and a controller that: controls the X-rays source to image a portion of the patient's body that passes through the FOV prior to the ROI with X-rays at a first intensity; estimates an entry position of the couch for which the ROI is expected to enter the FOV from at least one feature in the prior portion image; determines an ROI imaging start position for the couch, responsive to the estimated entry position; and controls the X-ray source to illuminate the FOV with X-rays at a second intensity suitable for imaging the ROI when the couch reaches the ROI imaging start position to initiate imaging of the ROI.

Optionally, the controller estimates an exit position of the couch at which the ROI is expected to exit the FOV. Optionally, the controller controls intensity of X-rays provided by the X-ray source responsive to the estimated exit position. Optionally, controlling the intensity of X-rays responsive to the exit position comprises reducing intensity of X-rays provided by the X-ray source. Optionally, reducing the intensity comprises shutting off the X-ray source.

In some embodiments of the present invention, the controller estimates a size of the patient's body responsive to the at least one feature in the image of the portion to estimate the exit position.

In some embodiments of the present invention, the controller estimates a size of the ROI responsive to the at least one feature in the image of the ROI to estimate the exit position.

In some embodiments of the present invention, the controller estimates a size of the patient's body responsive to the at least one feature in the image of the portion to estimate the entry position.

In some embodiments of the present invention, the CT scanner comprises a data base having known anatomical data that provides relative location and/or size of features of the human body. Optionally, a position that the controller estimates, it estimates using anatomical data from the database.

Optionally the CT scanner comprises a data base having personal data particular to the patient. Optionally, a position that the controller estimates, it estimates using personal data from the database.

In some embodiments of the present invention, the CT scanner comprises an expert program and a position that the controller estimates, it estimates using the expert program.

In some embodiments of the present invention, the controller controls the X-ray source to perform a planar scan to illuminate the FOV at the first intensity.

In some embodiments of the present invention, the controller controls the X-ray source to perform an axial scan to illuminate the FOV at the first intensity.

In some embodiments of the present invention, the controller controls the X-ray source to perform an axial scan to illuminate the FOV at the second intensity.

In some embodiments of the present invention, the controller controls the X-ray source to perform a helical scan to illuminate the FOV at the first intensity.

In some embodiments of the present invention, the controller controls the X-ray source to perform a helical scan to illuminate the FOV at the second intensity.

In some embodiments of the present invention, the first intensity is less than the second intensity.

There is further provided in accordance with an embodiment of the present invention, a method of controlling a CT scanner having a field of view (FOV) and an X-ray source that illuminates the FOV with X-rays to image a region of interest (ROI) of a patient comprising: moving the patient through the FOV; illuminating the FOV with X-rays at a first intensity to acquire an image of a portion of the patient's body that passes through the FOV prior to the ROI; estimating, responsive to at least one feature in the image of the portion, an entry position for the patient at which, during motion of the patient through the FOV, the ROI is expected to enter the FOV; controlling the X-ray source to illuminate the FOV with X-rays at a second intensity suitable for imaging the ROI at a position of the patient responsive to the entry position; and continuing thereafter illumination of the FOV with X-rays at the second intensity to acquire an image of the ROI.

Optionally the method comprises: estimating an exit position of the patient at which the ROI is expected to exit the FOV; determining an ROI imaging stop position responsive to the estimated exit position; and controlling intensity of X-rays provided by the X-ray source at a position of the patient responsive to the exit position.

Optionally, controlling the intensity of X-rays responsive to the exit position comprises reducing intensity of X-rays provided by the X-ray source. Optionally reducing the intensity comprises shutting off the X-ray source.

In some embodiments of the present invention, estimating the exit position comprises estimating a size of the patient's body responsive to the at least one feature in the image of the portion.

In some embodiments of the present invention, estimating the exit position comprises estimating a size of the ROI responsive to the at least one feature in the image of the ROI.

In some embodiments of the present invention, estimating the exit position comprises using known anatomical data that provides relative locations and/or sizes of features of the human body. Optionally, estimating the exit position comprises using personal data particular to the patient on which the anatomical data may depend.

In some embodiments of the present invention, estimating the exit position comprises using an expert program.

In some embodiments of the present invention, estimating the entry position comprises displaying the image of the portion and estimating the position responsive to visual inspection of the image.

In some embodiments of the present invention, estimating the entry position comprises estimating a size of the patient's body responsive to the at least one feature in the image of the portion.

In some embodiments of the present invention, estimating the entry position comprises using known anatomical data that provides relative locations and/or sizes of features of the human body. Optionally, estimating the entry position comprises using personal data particular to the patient on which the anatomical data may depend.

In some embodiments of the present invention, estimating the entry position comprises using an expert program.

In some embodiments of the present invention, estimating the entry position comprises displaying the image of the portion and estimating the position responsive to visual inspection of the image.

In some embodiments of the present invention, controlling the X-ray source to illuminate the FOV comprises manually controlling the X-ray source.

In some embodiments of the present invention, controlling the X-ray source to illuminate the FOV comprises automatically controlling the X-ray source.

In some embodiments of the present invention, controlling the X-ray source to illuminate the FOV at the first intensity comprises controlling the X-ray source to illuminate the FOV in a planar scan.

In some embodiments of the present invention, controlling the X-ray source to illuminate the FOV at the second intensity comprises controlling the X-ray source to illuminate the FOV in an axial scan.

In some embodiments of the present invention, controlling the X-ray source to illuminate the FOV at the first intensity comprises controlling the X-ray source to illuminate the FOV in a helical scan.

In some embodiments of the present invention, the first intensity is less than the second intensity.

### BRIEF DESCRIPTION OF FIGURES

Non-limiting examples of embodiments of the present invention are described below with reference to figures attached hereto. In the figures, identical structures, elements or parts that appear in more than one figure are generally labeled with a same numeral in all the figures in which they appear. Dimensions of components and features shown in the figures are chosen for convenience and clarity of presentation and are not necessarily shown to scale. The figures are listed below.
Fig. 1 schematically shows a multislice CT scanner 20 for imaging patients, in accordance with an embodiment of the present invention; and
Fig. 2 is a flow diagram of an algorithm responsive to which a controller controls the CT scanner shown in Fig. 1, in accordance with an embodiment of the present invention.

### DETAILED DESCRIPTION OF EXEMPLARY EMBODIMENTS

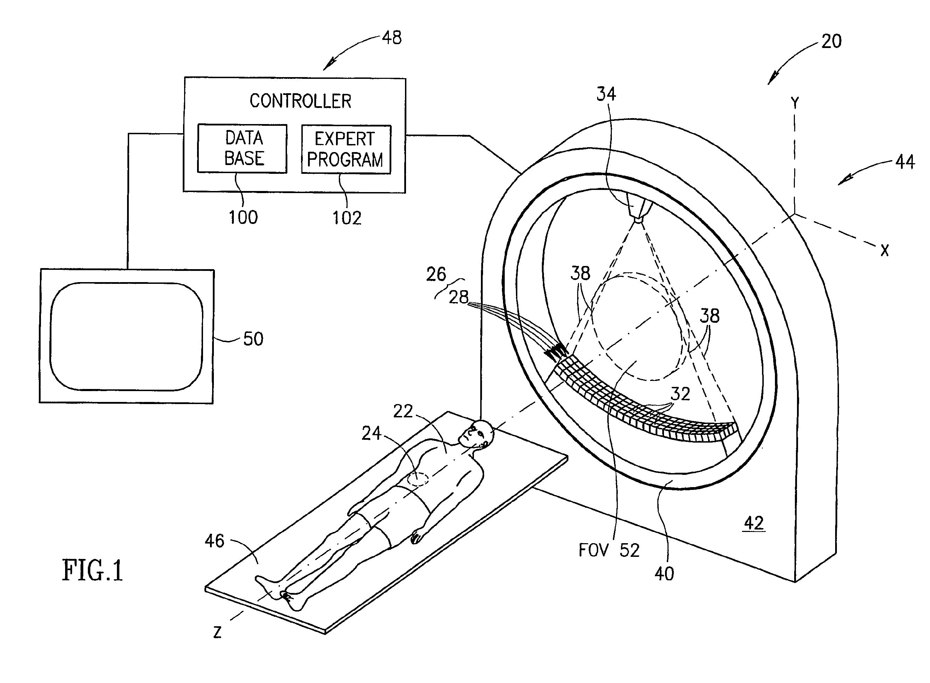

Fig. 1 schematically shows a third generation multislice CT scanner 20 for imaging patients, in accordance with an embodiment of the present invention. In Fig. 1 a patient 22 having an ROI 24 shown in dashed lines is to be imaged with scanner 20. ROI 24 is by way of example, a region of the patient's liver. Only features of multislice scanner 20 germane to the discussion are shown in Fig. 1.

Multislice scanner 20 comprises a detector array 26 having arcuate rows 28 of X-ray detectors 32 and an X-ray source 34 that provides a cone beam of X-rays shown in dashed lines 38 for illuminating patient 22 with X-rays. X-ray source 34 and detector array 26 are mounted to a rotor 40 of a gantry 42 comprised in scanner 20. Rotor 40 is rotatable around the z-axis of a coordinate system 44. By way of example, detector array 26 is shown comprising four rows 28 of detectors 32.

A field of view 52, *i.e.* FOV 52, of scanner 20 is located between X-ray source 34 and detector array 26 and is indicated by dashed lines. Patient 22 is supported on a couch 46 during imaging of the patient. Couch 46 is controllable to be translated axially along the z-axis to move patient 22 and ROI 24 through FOV 52. A controller 48 controls motion of couch 46, rotor 40 and intensity of X-rays provided by X-ray source 34. The controller optionally comprises a database 100 and an expert program 102 discussed below. Scanner 20 optionally comprises a video display console 50 on which images of patient 24 generated by scanner 20 are displayed.

Multislice scanner 20 can generally be operated in an axial mode or in a helical mode to image ROI 24 of patient 22. In an axial mode controller 48 controls motion of couch 46 to step patient 22 along the z-axis through FOV 52 of scanner 20. Following each step, rotor 40 rotates around the z-axis to rotate X-ray source 34 and cone beam 38 around patient 22 so as to acquire attenuation measurements for imaging the patient from a plurality of view angles. In a helical mode, patient 22 is moved continuously along the z-axis through FOV 52 as rotor 40 simultaneously, continuously rotates around the z-axis to acquire attenuation measurements.

In prior art scanners, to image ROI 24 controller 48 moves patient 22 in either an axial, helical or planar mode through FOV 52 to acquire a reconnaissance scan of the patient. The reconnaissance scan provides data for planning an imaging scan of patient 22, which is performed after the reconnaissance scan.

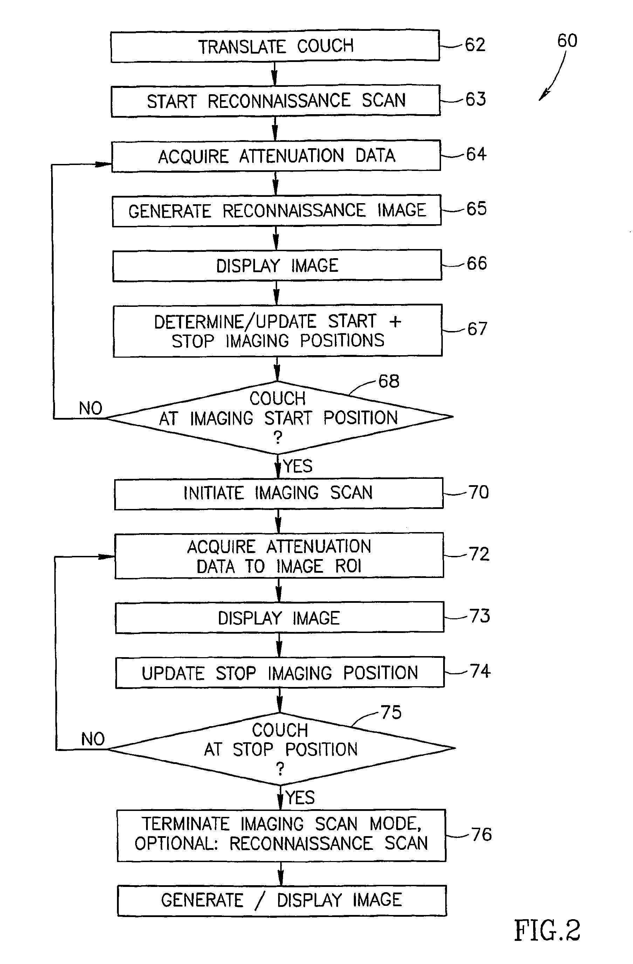

In accordance with an embodiment of the present invention, controller 48 controls scanner 20 to perform a dual function scan that comprises both an optionally low X-ray intensity reconnaissance mode scan and a relatively high X-ray intensity imaging mode scan. In some embodiments of the present invention, controller 48 controls scanner 20 to perform the dual function scan in accordance with an algorithm 60 comprising operations shown in a block diagram in Fig. 2. Scanner 20 may operate in either an axial scan mode or a helical scan mode during the imaging portion of a dual function scan. Generally, scanner 20 operates in an axial mode or a helical mode respectively in the reconnaissance portion of the dual function scan if the scanner operates in the axial mode or helical mode in the imaging portion of the dual function scan. However, for operation in either an axial or helical mode in the imaging portion of a dual function scan, scanner 20 may perform a reconnaissance scan in an axial mode in the reconnaissance portion of the dual function scan, in accordance with an embodiment of the invention. For convenience of presentation, for operation of scanner 20 in accordance with algorithm 60, it is assumed that the scanner is operating in a helical mode.

Referring to Fig. 2, after patient 22 is suitably accommodated on couch 46, in a block 62 of algorithm 60 controller 48 starts translating couch 46 along the z-axis to move patient 20 through FOV 52. In a block 63, at a z-coordinate, *i.e*. a reconnaissance start position, that is reached during translation of the couch prior to the entry of ROI 24 into FOV 52, controller 48 initiates a reconnaissance mode scan of patient 22. To initiate the reconnaissance mode scan controller 48 controls X-ray source 34 to generate an optionally low intensity X-ray cone beam and rotor 40 to rotate the X-ray source around the z-axis. Optionally, a reconnaissance start position of couch 46 is reached shortly before any part of patient 22 enters FOV 52.

Subsequent to reaching the reconnaissance start position, as patient 22 moves through FOV 52, in a block 64 controller 48 acquires attenuation measurements provided by detectors 32 of array 26 from an increasing portion of the body of patient 22. In a block 65, controller 48 processes the attenuation data to generate an image, *i.e.* a reconnaissance image, of the scanned portion of the body of patient 22. Optionally in a block 66 controller 48 displays the reconnaissance image on video console 50 (Fig. 1) so that an operator can monitor progress of the dual function scan. In a block 67 controller 48 processes the reconnaissance image to determine start and stop imaging positions of couch 46 (*i.e*. z-coordinates of the couch) at which to start and stop imaging patient 22.

To determine the imaging start and stop positions, controller 48 identifies and locates features in the reconnaissance image, hereinafter referred to as "fiducial features", relative to which a location for ROI 24 can be estimated. Controller 48 also optionally estimates a size of the body of patient 22 from the reconnaissance image that provides a scale of distances between features of the patient's body. The locations of the fiducial features and the estimated location of ROI 24 are optionally defined by coordinates relative to coordinate system 44 or a suitable alternative coordinate system. Controller 48 estimates imaging start and stop coordinates for ROI 24 using the coordinates of the fiducial features, the scale factor and known anatomical data that relates a position of ROI 24 to positions of the fiducial features.

The estimated coordinates of ROI 24 are used to estimate positions of couch 46 at which ROI 24 is expected to enter and exit FOV 52 and therefrom to determine imaging start and stop positions of the couch respectively. The imaging start and stop positions are determined so as to be located sufficiently displaced relative to the entry and exit positions at which ROI 24 is expected to enter and exit FOV 52 to assure that all of ROI 24 is properly imaged during an imaging scan mode of the dual function scan.

For example, controller 48 may count and locate vertebrae and/or ribs in the reconnaissance image and using the scale factor determined from the reconnaissance image determine from the located ribs and/or vertebrae where to expect location of the patient's liver, which by way of example is ROI 24. From the expected location of ROI 24 controller 48 determines at which positions of couch 46 the ROI is expected to enter and exit FOV 52 and therefrom imaging start and stop positions respectively. Optionally, organs identified and located in the reconnaissance image may be used as fiducial features to infer location of other organs in the patient's body and/or ROI 24. For example, an upper edge of the liver may serve as a fiducial feature to infer the location of the pancreas, or gall bladder.

In accordance with an embodiment of the present invention, controller 48 comprises a database 100 (Fig. 1) having personal data particular to patient 22 entered into the data base prior to imaging of the patient with scanner 20 and general anatomical and medical data. The controller uses data from the database to determine location of ROI 24. Personal data may include, for example, age, sex, height and weight of patient 22 whether patient 22 is a mesomorph or an ectomorph and medical history data such as whether patient 22 exercises regularly or is a heavy drinker. General medical data may include statistical data useable to correlate a location of ROI 24 with a characteristic of ROI 24, such as its identity as a particular organ, which in the case of the present example is the liver, or region of an organ. General medical data may also include statistical data useable to correlate a location of ROI 24 with personal data of patient 22 and data generated from the reconnaissance image of the patient. In some embodiments of the present invention, database 100 is constantly updated with data from each patient imaged by scanner 20 so that the database evolves and becomes more comprehensive with time.

In some embodiments of the present invention, controller 48 comprises and operates an expert program 102 (Fig. 1) to process data from the reconnaissance image and database 100 and determine suitable imaging start and stop positions. In some embodiments of the present invention, the expert program evolves and improves with accumulation of data by the database. In some embodiments of the present invention, database 100 shares with and incorporates data from a database of another CT scanner similar to CT scanner 20.

In a block 68 controller 48 compares the imaging start position with the couch. If the couch has not yet reached the imaging start position the controller optionally returns to block 64. In block 64 controller 48 acquires additional attenuation data in the reconnaissance scan mode and proceeds through block 65 to block 67 to update the reconnaissance image and the imaging start and stop positions. The controller returns again to block 68 to compare the updated start position with the couch position. Controller 48 continuously cycles through blocks 64 to 68 to determine if the couch position is substantially equal to the start position.

If the couch position is substantially equal to the imaging start position, controller 48 advances to a block 70. In block 70 controller 48 adjusts intensity of X-rays provided by X-ray source 34 to an intensity suitable for imaging ROI 24 and initiates an imaging mode scan of the dual function scan of patient 22. In a block 72 controller 48 acquires attenuation data for imaging ROI 24. In a block 73 controller 48 optionally combines attenuation data acquired for imaging ROI 24 and reconnaissance attenuation data and generates from the combined data an image which the controller displays on video console 50 to indicate progress of the dual function scan to an operator. Optionally, in a block 74 controller 48 uses the acquired imaging data to update the estimated couch position at which ROI 24 exits FOV 52 and therefrom the imaging stop position.

Controller 48 proceeds to a block 75 in which it compares the couch position to the imaging stop position. If the couch has not reached the imaging stop position, controller 48 returns to block 72 and acquires additional attenuation data for imaging ROI 24, optionally updates the imaging stop position in block 74 and compares the updated stop position with the couch position. If the couch position is substantially equal to the imaging stop position controller 48 proceeds to a block 76.

In block 76 controller 48 terminates the imaging scan mode of the dual function scan. In terminating the imaging scan mode the controller 76 shuts off X-ray source 34 or reduces intensity of X-rays from the X-ray source and controls scanner 20 to continue scanning patient 22 in a reconnaissance mode for a short period of time before shutting off the X-ray source. In a block 78 controller 48 optionally generates an image of ROI 24 and displays the image on video console 50.

Whereas, in accordance with algorithm 60 scanner 20 performs a dual function scan of a patient automatically, in some embodiments of the present invention an operator of the scanner may override operations performed in accordance with the algorithm and manually control the dual function scan.

For example, in some embodiments of the present invention an operator may take over control of a dual function scan at blocks 67-70 and determine and implement an imaging start position responsive to a reconnaissance image displayed by controller 48 in block 66. Similarly, the operator may determine an imaging stop position from an image displayed by controller 48 in step 73 and thereafter usurp the controller in determining when to terminate the imaging scan mode. In some embodiments of the present invention when an operator overrides controller 48, the controller presents the operator with visual or audio cues to alert the operator as to when couch positions for which the controller expects ROI 24 to enter and exit FOV 52 will be reached. For example, controller 48 may display a block arrow on an image displayed in block 66 that points in a direction of ROI 24 in the image and has a length that shortens as the couch approaches an estimated imaging start position. Alternatively, a color of the arrow may change for example from red to yellow to green to cue the operator, similarly to the way in which traffic light color changes cue a driver. Additionally or alternatively a warning beep repeated with increasing frequency as a start or stop position is approached may be sounded. Variations of algorithm 60 and operator override formats of the algorithm will occur to a person of the art.

It is noted that whereas in the description above of a CT scanner in accordance with the present invention, the scanner is controlled responsive to positions of the couch, in some embodiments of the invention the scanner is controlled responsive to corresponding times at which the positions of the couch are reached. For example the CT scanner may employ a clock and use couch translation speed to determine a time, an "imaging start time", at which an imaging start position of the couch is estimated to be reached and control the scanner responsive to the imaging start time.

In the description and claims of the present application, each of the verbs, "comprise" "include" and "have", and conjugates thereof, are used to indicate that the object or objects of the verb are not necessarily a complete listing of members, components, elements or parts of the subject or subjects of the verb.

The present invention has been described using detailed descriptions of embodiments thereof that are provided by way of example and are not intended to limit the scope of the invention. The described embodiments comprise different features, not all of which are required in all embodiments of the invention. Some embodiments of the present invention utilize only some of the features or possible combinations of the features. Variations of embodiments of the present invention that are described and embodiments of the present invention comprising different combinations of features noted in the described embodiments will occur to persons of the art. The scope of the invention is limited only by the following claims.

## Claims

1. A CT scanner for imaging an ROI of a patient comprising:
an X-ray source and detector array that define an FOV of the scanner;
a couch on which the patient is supported that moves the patient through the FOV; and a controller that:
controls the X-rays source to image a portion of the patient's body that passes through the FOV prior to the ROI with X-rays at a first intensity;
estimates an entry position of the couch for which the ROI is expected to enter the FOV from at least one feature in the prior portion image;
determines a ROI imaging start position for the couch, responsive to the estimated entry position; and
controls the X-ray source to illuminate the FOV with X-rays at a second intensity suitable for imaging the ROI when the couch reaches the ROI imaging start position to initiate imaging of the ROI.

2. A CT scanner according to claim 1 wherein the controller estimates an exit position of the couch at which the ROI is expected to exit the FOV.

3. A CT scanner according to claim 2 wherein the controller controls intensity of X-rays provided by the X-ray source responsive to the estimated exit position.

4. A CT scanner according to claim 3 wherein controlling the intensity of X-rays responsive to the exit position comprises reducing intensity of X-rays provided by the X-ray source.

5. A CT scanner according to claim 4 wherein reducing the intensity comprises shutting off the X-ray source.

6. A CT scanner according to any of claims 2-5 wherein the controller estimates a size of the patient's body responsive to the at least one feature in the image of the portion to estimate the exit position.

7. A CT scanner according to any of claims 2-6 wherein the controller estimates a size of the ROI responsive to the at least one feature in the image of the ROI to estimate the exit position.

8. A CT scanner according to any of claims 1-7 wherein the controller estimates a size of the patient's body responsive to the at least one feature in the image of the portion to estimate the entry position.

9. A CT scanner according to any of claims 1-8 comprising a data base having know anatomical data that provides relative location and/or size of features of the human body.

10. A CT scanner according to claim 9 wherein a position that the controller estimates, it estimates using anatomical data from the database.

11. A CT scanner according to claim 10 and comprising a data base having personal data particular to the patient.

12. A CT scanner according to claim 11 wherein the controller estimates a position using personal data from the database.

13. A CT scanner according to any of claims 1-11 wherein the controller comprises an expert program and wherein the controller estimates a position using the expert program.

14. A CT scanner according to any of claims 1-13 wherein the controller controls the X-ray source to perform a planar scan to illuminate the FOV at the first intensity.

15. A CT scanner according to any of claims 1-13 wherein the controller controls the X-ray source to perform an axial scan to illuminate the FOV at the first intensity.

16. A CT scanner according to any of claims 1-15 wherein the controller controls the X-ray source to perform an axial scan to illuminate the FOV at the second intensity.

17. A CT scanner according to any of claims 1-13 wherein the controller controls the X-ray source to perform a helical scan to illuminate the FOV at the first intensity.

18. A CT scanner according to any of claims 1-14 or claim 17 wherein the controller controls the X-ray source to perform a helical scan to illuminate the FOV at the second intensity.

19. A CT scanner according to any of claims 1-18 wherein the first intensity is less than the second intensity.

20. A method of controlling a CT scanner having a field of view (FOV) and an X-ray source that illuminates the FOV with X-rays to image a region of interest (ROI) of a patient comprising:
moving the patient through the FOV;
illuminating the FOV with X-rays at a first intensity to acquire an image of a portion of the patient's body that passes through the FOV prior to the ROI;
estimating, responsive to at least one feature in the image of the portion, an entry position for the patient at which, during motion of the patient through the FOV, the ROI is expected to enter the FOV ;
controlling the X-ray source to illuminate the FOV with X-rays at a second intensity suitable for imaging the ROI at a position of the patient responsive to the entry position; and
continuing thereafter illumination of the FOV with X-rays at the second intensity to acquire an image of the ROI.

21. A method according to claim 20 and comprising:
estimating an exit position of the patient at which the ROI is expected to exit the FOV;
determining an ROI imaging stop postion responsive to the estimated exit position; and
controlling intensity of X-rays provided by the X-ray source at a position of the patient responsive to the exit position.

22. A method according to claim 21 wherein controlling the intensity of X-rays responsive to the exit position comprises reducing intensity of X-rays provided by the X-ray source.

23. A method according to claim 22 wherein reducing the intensity comprises shutting off the X-ray source.

24. A method according to any of claims 21-23 wherein estimating the exit position comprises estimating a size of the patient's body responsive to the at least one feature in the image of the portion.

25. A method according to any of claims 21-24 wherein estimating the exit position comprises estimating a size of the ROI responsive to the at least one feature in the image of the ROI.

26. A method according to any of claims 21-25 wherein estimating the exit position comprises using known anatomical data that provides relative locations and/or sizes of features of the human body.

27. A method according to claim 26 wherein estimating the exit position comprises using personal data particular to the patient on which the anatomical data may depend.

28. A method according to any of claims 21-27 wherein estimating the exit position comprises using an expert program.

29. A method according to any of claims 20-27 wherein estimating the entry position comprises displaying the image of the portion and estimating the position responsive to visual inspection of the image.

30. A method according to any of claims 20-29 wherein estimating the entry position comprises estimating a size of the patient's body responsive to the at least one feature in the image of the portion.

31. A method according to any of claims 20-30 wherein estimating the entry position comprises using known anatomical data that provides relative locations and/or sizes of features of the human body.

32. A method according to claim 31 wherein estimating the entry position comprises using personal data particular to the patient on which the anatomical data may depend.

33. A method according to any of claims 20-32 wherein estimating the entry position comprises using an expert program.

34. A method according to any of claims 20-33 wherein estimating the entry position comprises displaying the image of the portion and estimating the position responsive to visual inspection of the image.

35. A method according to any of claims 20-34 wherein controlling the X-ray source to illuminate the FOV comprises manually controlling the X-ray source.

36. A method according to any of claims 20-34 wherein controlling the X-ray source to illuminate the FOV comprises automatically controlling the X-ray source.

37. A method according to any of claims 20-36 wherein controlling the X-ray source to illuminate the FOV at the first intensity comprises controlling the X-ray source to illuminate the FOV in a planar scan.

38. A method according to any of claims 20-36 wherein controlling the X-ray source to illuminate the FOV at the second intensity comprises controlling the X-ray source to illuminate the FOV in an axial scan.

39. A method according to any of claims 20-36 wherein controlling the X-ray source to illuminate the FOV at the first intensity comprises controlling the X-ray source to illuminate the FOV in a helical scan.

40. A method according to any of claims 20-39 wherein the first intensity is less than the second intensity.

## Patentansprüche

1. CT-Scanner zur Abbildung eines ROI eines Patienten, mit:
einer Röntgenstrahlenquelle und Detektoranordnung, die ein FOV des Scanners definieren;
einer Liege, auf welcher der Patient getragen wird und die den Patienten durch das FOV bewegt; sowie
einer Steuereinheit, welche:
die Röntgenstrahlenquelle steuert, um vor dem ROI einen das FOV passierenden Abschnitt des Körpers des Patienten mit Röntgenstrahlen bei einer ersten Intensität abzubilden;
anhand von mindestens einem Merkmal in der vorherigen Abschnittsabbildung eine Eintrittsposition der Liege berechnet, bei der das Eintreten des ROI in das FOV erwartet wird;
in Reaktion auf die berechnete Eintrittsposition eine ROI-Abbildungsstartposition für die Liege ermittelt; und
die Röntgenstrahlenquelle steuert, um das FOV mit Röntgenstrahlen bei einer zweiten Intensität zu beleuchten, die geeignet ist, um den ROI abzubilden, sobald die Liege die Abbildungsstartposition erreicht, um einen Abbildungsvorgang des ROI auszulösen.

2. CT-Scanner nach Anspruch 1, wobei die Steuereinheit eine Austrittsposition der Liege berechnet, bei welcher erwartet wird, dass der ROI das FOV verlässt.

3. CT-Scanner nach Anspruch 2, wobei die Steuereinheit die Intensität von Röntgenstrahlen steuert, die von der Röntgenstrahlenquelle in Reaktion auf die berechnete Austrittsposition vorgesehen werden.

4. CT-Scanner nach Anspruch 3, wobei die Steuerung der Intensität von Röntgenstrahlen in Reaktion auf die Austrittsposition die Reduzierung der Intensität von, von der Röntgenstrahlenquelle vorgesehenen Röntgenstrahlen umfasst.

5. CT-Scanner nach Anspruch 4, wobei die Reduzierung der Intensität das Abschalten der Röntgenstrahlenquelle umfasst.

6. CT-Scanner nach einem der Ansprüche 2-5, wobei die Steuereinheit in Reaktion auf das mindestens eine Merkmal in der Abbildung des Abschnitts eine Größe des Körpers des Patienten berechnet, um die Austrittsposition zu berechnen.

7. CT-Scanner nach einem der Ansprüche 2-6, wobei die Steuereinheit in Reaktion auf das mindestens eine Merkmal in der Abbildung des ROI eine Größe des ROI berechnet, um die Austrittsposition zu berechnen.

8. CT-Scanner nach einem der Ansprüche 1-7, wobei die Steuereinheit in Reaktion auf das mindestens eine Merkmal in der Abbildung des Abschnitts eine Größe des Körpers des Patienten berechnet, um die Eintrittsposition zu berechnen.

9. CT-Scanner nach einem der Ansprüche 1-8, der eine Datenbank mit bekannten anatomischen Daten aufweist, die eine relative Lage und/oder Größe von Merkmalen des menschlichen Körpers vorsehen.

10. CT-Scanner nach Anspruch 9, wobei eine Position, welche die Steuereinheit berechnet, unter Verwendung von anatomischen Daten aus der Datenbank berechnet wird.

11. CT-Scanner nach Anspruch 10, der eine Datenbank mit persönlichen Daten, speziell für den Patienten, aufweist.

12. CT-Scanner nach Anspruch 11, wobei die Steuereinheit eine Position unter Verwendung persönlicher Daten aus der Datenbank berechnet.

13. CT-Scanner nach einem der Ansprüche 1-11, wobei die Steuereinheit ein Expertenprogramm umfasst und eine Position unter Verwendung des Expertenprogramms berechnet.

14. CT-Scanner nach einem der Ansprüche 1-13, wobei die Steuereinheit die Röntgenstrahlenquelle steuert, um zur Beleuchtung des FOV bei der ersten Intensität einen planaren Scan durchzuführen.

15. CT-Scanner nach einem der Ansprüche 1-13, wobei die Steuereinheit die Röntgenstrahlenquelle steuert, um zur Beleuchtung des FOV bei der ersten Intensität einen axialen Scan durchzuführen.

16. CT-Scanner nach einem der Ansprüche 1-15, wobei die Steuereinheit die Röntgenstrahlenquelle steuert, um zur Beleuchtung des FOV bei der zweiten Intensität einen axialen Scan durchzuführen.

17. CT-Scanner nach einem der Ansprüche 1-13, wobei die Steuereinheit die Röntgenstrahlenquelle steuert, um zur Beleuchtung des FOV bei der ersten Intensität einen spiralförmigen Scan durchzuführen.

18. CT-Scanner nach einem der Ansprüche 1-14 oder Anspruch 17, wobei die Steuereinheit die Röntgenstrahlenquelle steuert, um zur Beleuchtung des FOV bei der zweiten Intensität einen spiralförmigen Scan durchzuführen.

19. CT-Scanner nach einem der Ansprüche 1-18, wobei die erste Intensität geringer als die zweite Intensität ist.

20. Verfahren zur Steuerung eines CT-Scanners mit einem Sichtfeld (FOV) und einer Röntgensstrahlenquelle, die das FOV mit Röntgenstrahlen beleuchtet, um einen interessierenden Bereich (ROI) eines Patienten abzubilden, wobei das Verfahren die folgenden Schritte umfasst:
Bewegen des Patienten durch das FOV;
Beleuchten des FOV mit Röntgenstrahlen bei einer ersten Intensität, um eine Abbildung eines Abschnitts des Körpers des Patienten zu erhalten, der das FOV vor dem ROI passiert;
Berechnen einer Eintrittsposition für den Patienten in Reaktion auf mindestens ein Merkmal in der Abbildung des Abschnitts, bei welcher, während der Patient durch das FOV bewegt wird, das Eintreten des ROI in das FOV erwartet wird;
Steuerung der Röntgenstrahlenquelle, um das FOV mit Röntgenstrahlen bei einer zweiten Intensität zu beleuchten, die geeignet ist, um den ROI bei einer Position des Patienten in Reaktion auf die Eintrittsposition abzubilden; sowie
anschließendes fortgesetztes Beleuchten des FOV mit Röntgenstrahlen bei der zweiten Intensität, um eine Abbildung des ROI zu erhalten.

21. Verfahren nach Anspruch 20, welches die folgenden Schritte umfasst:
Berechnen einer Austrittsposition des Patienten, bei welcher erwartet wird, dass der ROI das FOV verlässt;
Ermitteln einer Stop-Position der ROI-Abbildung in Reaktion auf die berechnete Austrittsposition; sowie
Steuerung der Intensität von, von der Röntgenstrahlenquelle vorgesehenen Röntgenstrahlen bei einer Position des Patienten in Reaktion auf die Austrittsposition.

22. Verfahren nach Anspruch 21, wobei die Steuerung der Intensität von Röntgenstrahlen in Reaktion auf die Austrittsposition die Reduzierung der Intensität von, von der Röntgenstrahlenquelle vorgesehenen Röntgenstrahlen umfasst.

23. Verfahren nach Anspruch 22, wobei die Reduzierung der Intensität das Abschalten der Röntgenstrahlenquelle umfasst.

24. Verfahren nach einem der Ansprüche 21-23, wobei das Berechnen der Austrittsposition das Berechnen einer Größe des Körpers des Patienten in Reaktion auf das mindestens eine Merkmal in der Abbildung des Abschnitts umfasst.

25. Verfahren nach einem der Ansprüche 21-24, wobei das Berechnen der Austrittsposition das Berechnen einer Größe des ROI in Reaktion auf das mindestens eine Merkmal in der Abbildung des ROI umfasst.

26. Verfahren nach einem der Ansprüche 21-25, wobei das Berechnen der Austrittsposition die Verwendung bekannter anatomischer Daten umfasst, die relative Lagen und/oder Größen von Merkmalen des menschlichen Körpers vorsehen.

27. Verfahren nach Anspruch 26, wobei das Berechnen der Austrittsposition die Verwendung persönlicher Daten, speziell für den Patienten, von denen die anatomischen Daten abhängen können, umfasst.

28. Verfahren nach einem der Ansprüche 21-27, wobei das Berechnen der Austrittsposition die Verwendung eines Expertenprogramms umfasst.

29. Verfahren nach einem der Ansprüche 20-27, wobei das Berechnen der Eintrittsposition das Darstellen der Abbildung des Abschnitts und Berechnen der Position in Reaktion auf visuelle Prüfung der Abbildung umfasst.

30. Verfahren nach einem der Ansprüche 20-29, wobei das Berechnen der Eintrittsposition das Berechnen einer Größe des Körpers des Patienten in Reaktion auf das mindestens eine Merkmal in der Abbildung des Abschnitts umfasst.

31. Verfahren nach einem der Ansprüche 20-30, wobei das Berechnen der Eintrittsposition die Verwendung bekannter anatomischer Daten umfasst, die relative Lagen und/oder Größen von Merkmalen des menschlichen Körpers vorsehen.

32. Verfahren nach Anspruch 31, wobei das Berechnen der Eintrittsposition die Verwendung persönlicher Daten, speziell für den Patienten, von denen die anatomischen Daten abhängen können, umfasst.

33. Verfahren nach einem der Ansprüche 20-32, wobei das Berechnen der Eintrittsposition die Verwendung eines Expertenprogramms umfasst.

34. Verfahren nach einem der Ansprüche 20-33, wobei das Berechnen der Eintrittsposition das Darstellen der Abbildung des Abschnitts und Berechnen der Position in Reaktion auf visuelle Prüfung der Abbildung umfasst.

35. Verfahren nach einem der Ansprüche 20-34, wobei die Steuerung der Röntgenstrahlenquelle zur Beleuchtung des FOV die manuelle Steuerung der Röntgenstrahlenquelle umfasst.

36. Verfahren nach einem der Ansprüche 20-34, wobei die Steuerung der Röntgenstrahlenquelle zur Beleuchtung des FOV die automatische Steuerung der Röntgenstrahlenquelle umfasst.

37. Verfahren nach einem der Ansprüche 20-36, wobei die Steuerung der Röntgenstrahlenquelle zur Beleuchtung des FOV bei der ersten Intensität die Steuerung der Röntgenstrahlenquelle zur Beleuchtung des FOV in einem planaren Scan umfasst.

38. Verfahren nach einem der Ansprüche 20-36, wobei die Steuerung der Röntgenstrahlenquelle zur Beleuchtung des FOV bei der zweiten Intensität die Steuerung der Röntgenstrahlenquelle zur Beleuchtung des FOV in einem axialen Scan umfasst.

39. Verfahren nach einem der Ansprüche 20-36, wobei die Steuerung der Röntgenstrahlenquelle zur Beleuchtung des FOV bei der ersten Intensität die Steuerung der Röntgenstrahlenquelle zur Beleuchtung des FOV in einem spiralförmigen Scan umfasst.

40. Verfahren nach einem der Ansprüche 20-39, wobei die erste Intensität geringer als die zweite Intensität ist.

## Revendications

1. Tomodensitomètre pour l'imagerie d'une région d'intérêt d'un patient comprenant :
une source radiographique et un ensemble de détecteur définissant un champ de vue du scanner ;
une table sur laquelle le patient est supporté qui déplace le patient à travers le champ de vue ; et un contrôleur qui :
contrôle la source radiographique pour imager une partie du corps du patient qui traverse le champ de vue avant la région d'intérêt avec des rayons X à une première intensité ;
estime une position d'entrée de la table pour laquelle la région d'intérêt doit entrer dans le champ de vue à partir d'au moins une caractéristique dans l'image de la partie précédente ;
détermine une position de début d'imagerie de région d'intérêt pour la table, en réponse à la position d'entrée estimée ; et
contrôle la source radiographique pour éclairer le champ de vue avec les rayons X à une seconde intensité adaptée pour l'imagerie de la région d'intérêt quand la table atteint la position de début d'imagerie de région d'intérêt pour débuter l'imagerie de la région d'intérêt.

2. Tomodensitomètre selon la revendication 1, dans lequel le contrôleur estime une position de sortie de la table dans laquelle la région d'intérêt doit quitter le champ de vue.

3. Tomodensitomètre selon la revendication 2, dans lequel le contrôleur contrôle l'intensité des rayons X fournis par la source radiographique en réponse à la position de sortie estimée.

4. Tomodensitomètre selon la revendication 3, dans lequel le contrôle de l'intensité des rayons X en réponse à la position de sortie comprend la réduction de l'intensité des rayons X fournis par la source radiographique.

5. Tomodensitomètre selon la revendication 4, dans lequel la réduction de l'intensité comprend l'arrêt de la source radiographique.

6. Tomodensitomètre selon une ou plusieurs des revendications 2 à 5, dans lequel le contrôleur estime la taille du corps du patient en réponse à la au moins une caractéristique dans l'image de la partie pour estimer la position de sortie.

7. Tomodensitomètre selon une ou plusieurs des revendications 2 à 6, dans lequel le contrôleur estime la taille de la région d'intérêt en réponse à la au moins une caractéristique dans l'image de la région d'intérêt pour estimer la position de sortie.

8. Tomodensitomètre selon une ou plusieurs des revendications 1 à 7, dans lequel le contrôleur estime la taille du corps du patient en réponse à la au moins une caractéristique dans l'image de la partie pour estimer la position d'entrée.

9. Tomodensitomètre selon une ou plusieurs des revendications 1 à 8, comprenant une base de données comportant des données anatomiques connues fournissant l'emplacement relatif et/ou la taille des caractéristiques du corps humain.

10. Tomodensitomètre selon la revendication 9, dans lequel le contrôleur estime une position en utilisant les données anatomiques à partir de la base de données.

11. Tomodensitomètre selon la revendication 10, comprenant une base de données comportant des données personnelles liées au patient.

12. Tomodensitomètre selon la revendication 11, dans lequel le contrôleur estime une position en utilisant les données personnelles à partir de la base de données.

13. Tomodensitomètre selon une ou plusieurs des revendications 1 à 11, dans lequel le contrôleur comprend un programme expert et dans lequel le contrôleur estime une position en utilisant le programme expert.

14. Tomodensitomètre selon une ou plusieurs des revendications 1 à 13, dans lequel le contrôleur contrôle la source radiographique pour réaliser un balayage planaire pour éclairer le champ de vue à la première intensité.

15. Tomodensitomètre selon une ou plusieurs des revendications 1 à 13, dans lequel le contrôleur contrôle la source radiographique pour réaliser un balayage axial pour éclairer le champ de vue à la première intensité.

16. Tomodensitomètre selon une ou plusieurs des revendications 1 à 15, dans lequel le contrôleur contrôle la source radiographique pour réaliser un balayage axial pour éclairer le champ de vue à la seconde intensité.

17. Tomodensitomètre selon une ou plusieurs des revendications 1 à 13, dans lequel le contrôleur contrôle la source radiographique pour réaliser un balayage hélicoïdal pour éclairer le champ de vue à la première intensité.

18. Tomodensitomètre selon une ou plusieurs des revendications 1 à 14 ou la revendication 17, dans lequel le contrôleur contrôle la source radiographique pour réaliser un balayage hélicoïdal pour éclairer le champ de vue à la seconde intensité.

19. Tomodensitomètre selon une ou plusieurs des revendications 1 à 18, dans lequel la première intensité est inférieure à la seconde intensité.

20. Procédé de commande d'un tomodensitomètre ayant un champ de vue et une source radiographique qui éclaire le champ de vue avec des rayons X pour imager une région d'intérêt d'un patient comprenant :
le déplacement du patient à travers le champ de vue ;
l'éclairage du champ de vue avec des rayons X à une première intensité pour acquérir une image d'une partie du corps du patient qui traverse le champ de vue avant la région d'intérêt ;
l'estimation, en réponse à au moins une caractéristique dans l'image de la partie, d'une position d'entrée pour le patient dans laquelle, pendant le mouvement du patient à travers le champ de vue, la région d'intérêt doit entrer dans le champ de vue ;
le contrôle de la source radiographique pour éclairer le champ de vue avec des rayons X à une seconde intensité adaptée pour l'imagerie de la région d'intérêt dans une position du patient en réponse à la position d'entrée ; et
la poursuite de l'éclairage du champ de vue avec des rayons X à la seconde intensité pour acquérir une image de la région d'intérêt.

21. Procédé selon la revendication 20, comprenant :
l'estimation d'une position de sortie du patient dans laquelle la région d'intérêt doit quitter le champ de vue ;
la détermination d'une position d'arrêt de l'imagerie de la région d'intérêt en réponse à la position de sortie estimée ; et
le contrôle de l'intensité des rayons X fournis par la source radiographique dans une position du patient en réponse à la position de sortie.

22. Procédé selon la revendication 21, dans lequel le contrôle de l'intensité des rayons X en réponse à la position de sortie comprend la réduction de l'intensité des rayons X fournis par la source radiographique.

23. Procédé selon la revendication 22, dans lequel la réduction de l'intensité comprend l'arrêt de la source radiographique.

24. Procédé selon une ou plusieurs des revendications 21 à 23, dans lequel l'estimation de la position de sortie comprend l'estimation de la taille du corps du patient en réponse à la au moins une caractéristique dans l'image de la partie.

25. Procédé selon une ou plusieurs des revendications 21 à 24, dans lequel l'estimation de la position de sortie comprend l'estimation de la taille de la région d'intérêt en réponse à la au moins une caractéristique dans l'image de la région d'intérêt.

26. Procédé selon une ou plusieurs des revendications 21 à 25, dans lequel l'estimation de la position de sortie comprend l'utilisation des données anatomiques connues qui fournissent les emplacements relatifs et/ou les tailles des caractéristiques du corps humain.

27. Procédé selon la revendication 26, dans lequel l'estimation de la position de sortie comprend l'utilisation des données personnelles particulières du patient dont les données anatomiques peuvent dépendre.

28. Procédé selon une ou plusieurs des revendications 21 à 27, dans lequel l'estimation de la position de sortie comprend l'utilisation d'un programme expert.

29. Procédé selon une ou plusieurs des revendications 20 à 27, dans lequel l'estimation de la position d'entrée comprend l'affichage de l'image de la partie et l'estimation de la position en réponse à l'inspection visuelle de l'image.

30. Procédé selon une ou plusieurs des revendications 20 à 29, dans lequel l'estimation de la position d'entrée comprend l'estimation de la taille du corps du patient en réponse à la au moins une caractéristique dans l'image de la partie.

31. Procédé selon une ou plusieurs des revendications 20 à 30, dans lequel l'estimation de la position d'entrée comprend l'utilisation des données anatomiques connues qui fournissent les emplacements relatifs et/ou les tailles des caractéristiques du corps humain.

32. Procédé selon la revendication 31, dans lequel l'estimation de la position d'entrée comprend l'utilisation des données personnelles particulières du patient dont les données anatomiques peuvent dépendre.

33. Procédé selon une ou plusieurs des revendications 20 à 32, dans lequel l'estimation de la position d'entrée comprend l'utilisation d'un programme expert.

34. Procédé selon une ou plusieurs des revendications 20 à 33, dans lequel l'estimation de la position d'entrée comprend l'affichage de l'image de la partie et l'estimation de la position en réponse à l'inspection visuelle de l'image.

35. Procédé selon une ou plusieurs des revendications 20 à 34, dans lequel le contrôle de la source radiographique pour éclairer le champ de vue comprend le contrôle manuel de la source radiographique.

36. Procédé selon une ou plusieurs des revendications 20 à 34, dans lequel le contrôle de la source radiographique pour éclairer le champ de vue comprend le contrôle automatique de la source radiographique.

37. Procédé selon une ou plusieurs des revendications 20 à 36, dans lequel le contrôle de la source radiographique pour éclairer le champ de vue à la première intensité comprend le contrôle de la source radiographique pour éclairer le champ de vue dans un balayage planaire.

38. Procédé selon une ou plusieurs des revendications 20 à 36, dans lequel le contrôle de la source radiographique pour éclairer le champ de vue à la seconde intensité comprend le contrôle de la source radiographique pour éclairer le champ de vue dans un balayage axial.

39. Procédé selon une ou plusieurs des revendications 20 à 36, dans lequel le contrôle de la source radiographique pour éclairer le champ de vue à la première intensité comprend le contrôle de la source radiographique pour éclairer le champ de vue dans un balayage hélicoïdal.

40. Procédé selon une ou plusieurs des revendications 20 à 39, dans lequel la première intensité est inférieure à la seconde intensité.
